# EUROPEAN PATENT APPLICATION

(11) **EP 4 782 010 A1**
(43) Date of publication of application: **29.07.2026**
(21) Application number: 24867526.6
(22) Date of filing: 20.09.2024
(51) Int. Cl.: A61K 47/68, A61K 38/07, A61P 35/00

(54) **DRUG FOR TREATING NON-HODGKIN LYMPHOMA, PHARMACEUTICAL COMBINATION AND USE THEREOF**

(30) Priority: 21.09.2023 CN 202311225386
(71) Applicant: Zhejiang Teruisi Pharmaceutical Inc., Huzhou, Zhejiang 313000 (CN)
(72) Inventor: HUANG, Kai, Huzhou, Zhejiang 313000 (CN); HUANG, Jingyao, Huzhou, Zhejiang 313000 (CN)
(74) Representative: Rückerl, Florian
(86) International application number: PCT/CN2024/119914
(87) International publication number: WO 2025/061121

(57) **Abstract**

The use of an anti-CD20 antibody-drug conjugate or a pharmaceutical combination thereof in the preparation of a drug for treating non-Hodgkin lymphoma. The pharmaceutical combination comprises the anti-CD20 antibody-drug conjugate and at least one therapeutic agent. Compared with existing clinical second-line standard therapies, combination therapy using the anti-CD20 antibody-drug conjugate or the pharmaceutical combination comprising same has a better therapeutic effect.

## Description

### CROSS-REFERENCE TO RELATED APPLICATIONS

This application claims the benefit of priority to the China Invention Patent Application No. CN 202311225386.1 filed on September 21, 2023, the entire contents of the aforementioned application are incorporated herein by reference.

### TECHNICAL FIELD

The present disclosure relates to the field of pharmaceutical and drug combination technology, and particularly relates to drugs, drug combination for treating Non-Hodgkin Lymphoma (NHL) and a use thereof.

### BACKGROUND

Non-Hodgkin Lymphoma (NHL) is the seventh most common type of cancer in the world, accounting for ~4% to 5% of new cancer cases and 3% to 4% of cancer-related deaths, affecting ~1.5 million people worldwide. NHL is a general term for a group of malignant proliferative diseases of the lymphatic system, including many types of lymphocytomas, which mainly originate from B lymphocytes (> 85%), and a few originate from T cells and natural killer cells. It is usually classified as indolent NHL (iNHL) and aggressive NHL (aNHL) according to the prognosis. Among them, follicular lymphoma (FL) is the most common iNHL, accounting for ~1/5 of all NHL patients, while diffuse large B-cell lymphoma (DLBCL) is the most common aNHL, accounting for ~1/3 of all NHL patients.

In DLBCL, 30% to 35% of cases express MYC protein, and 20% to 35% co-express BCL2, referred to as "Double expressor lymphoma" (DEL), which indicates poor prognosis.

Enhancing therapeutic efficacy through drug combinations is a conventional approach in this field. However, clinically, standard combination regimens vary for different lines of therapy and subtypes of NHL. There are even more investigational combination regimens under study, and the number of available chemical and biological drugs for combination is even more extensive. This presents significant challenges in designing optimal drug combination regimens, with considerable uncertainty regarding their efficacy and safety.

WO2021223048A1 and the Chinese patent family CN115485304A disclose an anti-CD20 antibody drug conjugate (ADC), and mention in the description that the conjugate can be used in combination with Doxorubicin, Cyclophosphamide, taxanes, Capecitabine, Gemcitabine, Irinotecan, Oxaliplatin, Prednisone, Vincristine, etc., or a combination thereof. The earlier applications CN108452318A and CN108452319A of the present disclosure disclose an anti-CD20 ADC and a preparation thereof, respectively, and mention in the description that the ADC can be used in combination with Cyclophosphamide, Doxorubicin, Vincristine, Prednisone, PD1 antibody, CTLA4 inhibitor, or a combination thereof.

However, the above-mentioned patent application only lists the combination of anti-CD20 conjugate and commonly used anti-tumor drugs in clinical practice. The example does not design a specific drug combination regimen for the treatment of NHL and does not obtain efficacy and safety data. In addition, the above-mentioned taxanes, PD1 antibody, CTLA4 inhibitor, and other drugs used in combination with the anti-CD20 ADC are not even used in the treatment of NHL in clinical practice. The listing of various drugs used in combination in the above-mentioned patent is only one writing mode and is an accidental anticipation. Due to the wide variety of drugs available for combination, the large number of combination regimens, and the uncertainty of drug combination efficacy and combined drug toxicity superposition, the above-mentioned patent application cannot provide technical teaching for specific drug combination regimens.

The first-line standard treatment regimens for DLBCL currently recommended in the Guidelines for Diagnosis and Treatment of Lymphoma (2022 Edition) by the Chinese Society of Clinical Oncology (CSCO) are shown in Table a, including R-CHOP regimen, R-CHOEP regimen, R-miniCHOP regimen, DA-EPOCH-R regimen, and Pola-R-CHP regimen. The R-CHOP regimen uses a combination of Rituximab (R), Cyclophosphamide (C), Vincristine (H), Doxorubicin (O), and Prednisone (P); the R-CHOEP regimen uses a combination of Rituximab (R), Cyclophosphamide (C), Vincristine (H), Doxorubicin (O), Etoposide (E), and Prednisone (P); the R-miniCHOP regimen uses a combination of Rituximab (R), Cyclophosphamide (C), Vincristine (H), Doxorubicin (O), and Prednisone (P), with CHOP at a low dose; the DA-EPOCH-R regimen uses a combination of Etoposide (E), Prednisone (P), Doxorubicin (O), Cyclophosphamide (C), Vincristine (H), and Rituximab (R), and a dose adjustment (DA) is performed according to the patient's response to chemotherapy after each chemotherapy cycle; and the Pola-R-CHP regiment uses a combination of Polatuzumab vedotin (ADC targeting CD79b, trade name: Polivy^{®}), Rituximab (R), Cyclophosphamide (C), Vincristine (H), and Prednisone (P).

**Table a First-line Treatment Regimen for DLBCL**

| **First-line Treatment Regimen** |
|---|
| [R-CHOP] Rituximab + Cyclophosphamide + Doxorubicin + Vincristine + Prednisone |
| [R-CHOEP] Rituximab + Cyclophosphamide + Doxorubicin + Vincristine + Etoposide + Prednisone |
| [R-miniCHOP] Rituximab + reduced dose CHOP (dose reduced to 1/2 to 1/3 of the standard dose) |
| [DA-EPOCH-R] Rituximab + Etoposide + Prednisone + Vincristine + Cyclophosphamide + Doxorubicin |
| [Pola-R-CHP] Rituximab + Polatuzumab Vedotin + Cyclophosphamide + Doxorubicin + Prednisone |

Although patients have achieved varying degrees of relief after the above-mentioned first-line treatment, 40% to 50% are still prone to relapsed or refractory (R/R), especially for "DEL", which shows a poor prognosis.

The second-line standard treatment regimens for relapsed or refractory (R/R) diffuse large B-cell lymphoma (DLBCL) after treatment with the first-line standard treatment regimens currently recommended in the Guidelines for Diagnosis and Treatment of Lymphoma (2022 Edition) by the Chinese Society of Clinical Oncology (CSCO) is shown in Table b below, including R-GemOx (a combination of Rituximab (R), Gemcitabine (Gem) and Oxaliplatin (Ox)), R² (a combination of Rituximab (R) and Lenalidomide (R)), BR (a combination of Bendamustine (B) and Rituximab (R)), R-ICE (a combination of Rituximab, Ifosfamide, Carboplatin and Etoposide), R-DHAP (a combination of Rituximab, Cisplatin, Cytarabine and Dexamethasone), R-GDP (a combination of Rituximab, Gemcitabine, Cisplatin and Dexamethasone), R-MINE (a combination of Rituximab, Mesna, Ifosfamide, Mitoxantrone and Etoposide), R-ESHAP (a combination of Rituximab, Etoposide, Methylprednisolone, Cisplatin and Cytarabine), DA-EPOCH-R (a combination of Rituximab, Etoposide, Prednisone, Vincristine, Cyclophosphamide and Doxorubicin). The above-mentioned various regimens recommended by the Guidelines, such as the BR regimen, are also used as standard treatment regimens for other subtypes of NHL such as mantle cell lymphoma (MCL), follicular lymphoma (FL), and marginal zone lymphoma (MZL).

**Table b Second-line Treatment Regimen for DLBCL**

| **Second-line Treatment Regimen** |
|---|
| [R-DHAP] Rituximab + Cisplatin + Cytarabine + Dexamethasone |
| [R-ICE] Rituximab + Ifosfamide + Carboplatin + Etoposide |
| [R-GDP] Rituximab + Gemcitabine + Cisplatin + Dexamethasone |
| [R-ESHAP] Rituximab + Etoposide + Methylprednisolone + Cisplatin + Cytarabine |
| [DA-EPOCH-R] Rituximab + Etoposide + Prednisone + Vincristine + Cyclophosphamide + Doxorubicin |
| [R-GemOx] Rituximab + Gemcitabine + Oxaliplatin |
| [R-MINE] Rituximab + Mesna + Ifosfamide + Mitoxantrone + Etoposide |
| [R²] Rituximab + Lenalidomide |
| [BR] Rituximab + Bendamustine |

Although the patient has achieved a certain degree of remission after the above-mentioned second-line standard treatment regimen, there is still room for further improvement in clinical benefits. Therefore, it is still necessary to find more effective treatment or combination therapy drugs and treatment plans for NHL compared with existing standard treatments.

### SUMMARY

The purpose of the present disclosure is to provide a drug or drug combination and a use thereof in the preparation of a drug for treating Non-Hodgkin Lymphoma (NHL). The monotherapy or drug combination therapy can provide better efficacy than the existing clinical second-line standard therapy.

**A first aspect of the present disclosure provides a use of an anti-CD20 antibody drug conjugate (ADC) in the preparation of a drug for treating NHL.**

The anti-CD20 ADC has the following structure:

mAb-(L-D)n;

Wherein, mAb represents a recombinant anti-CD20 monoclonal antibody (mAb), D represents a small molecule toxin; L is a linker connecting the mAb and the small molecule toxin; n is the mean of small molecule toxins coupled to the mAb; and "-" is a bond.

Preferably, the recombinant anti-CD20 mAb includes but is not limited to Rituximab or a biosimilar thereof, Ofatumumab or a biosimilar thereof, Obinutuzumab or a biosimilar thereof, or Ocrelizumab or a biosimilar thereof.

More preferably, the recombinant anti-CD20 mAb is preferred to Rituximab or a biosimilar thereof.

Preferably, D is one or more monomethyl auristatins, including but not limited to MMAE, MMAD, or MMAF; further, the monomethyl auristatin is MMAE.

L is a cleavable linker or a non-cleavable linker. The non-cleavable linker includes but is not limited to a thioether bond linker comprising a maleimide group; preferably, the maleimide group comprises hexanoic acid to form maleimidocaproyl (mc); preferably, the maleimide group comprises a maleimidomethyl group, such as succinimidyl 4-(N-maleimidomethyl)cyclohexane-1-carboxylate (sMCC) or sulfo-sMCC.

The cleavable linker includes but is not limited to a hydrazone bond linker, a disulfide bond linker, and a peptide bond linker; the peptide bond linker comprises valine-citrulline (Val-Cit or VC), phenylalanine-lysine (Phe-Lys), valine-alanine (Val-Ala), or valine-lysine (Val-Lys). Preferably, the peptide bond linker is Val-Cit.

Further, the linker also contains para-aminobenzyl alcohol (PAB), para-aminobenzyl carbonate (PABC), or a derivative or analog thereof. Further, L is MC-VC-PAB.

The n is an integer or non-integer from 1 to 8; preferably, n is 4.2 ± 1, more preferably, 4.2 ± 0.5, and still more preferably, 4.2 ± 0.3.

The anti-CD20 ADC further has the following structure:

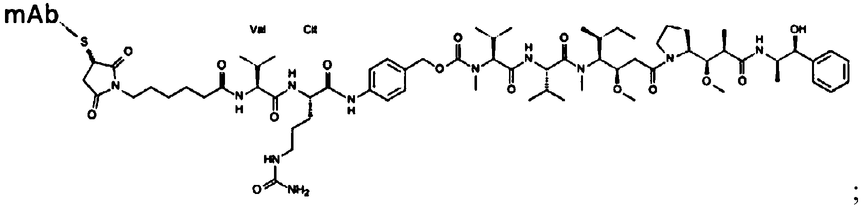

Wherein, mAb is Rituximab or a biosimilar thereof, n is the mean number of small molecule toxins coupled to the mAb and is 1 to 8; preferably, n is 4.2 ± 1, further preferably, n is 4.2 ± 0.5, and still more preferably, n is 4.2 ± 0.3.

The anti-CD20 ADC described in the present disclosure can be a pharmaceutical preparation containing an anti-CD20 ADC and a carrier or excipient, and the pharmaceutical preparation is a liquid preparation or a freeze-dried preparation.

The carrier or excipient is selected from the group consisting of a pH buffer, an osmotic pressure regulator, a lyophilized powder excipient, a protein protecting agent, a solubilizer, and water for injection (WFI).

The pH buffer includes histidine hydrochloride buffer, acetic acid buffer, phosphate buffer, citric acid buffer, or Tris buffer; preferably, the pH buffer is histidine hydrochloride buffer.

The protein protecting agent includes trehalose, sucrose, lactose, or glucose; preferably, the protein protecting agent is trehalose.

The osmotic pressure regulator includes mannitol or sodium chloride; preferably, the osmotic pressure regulator is mannitol.

The solubilizer includes polysorbate 80, polysorbate 20, or poloxamer 188; preferably, the solubilizer is polysorbate 80.

Further, the liquid or freeze-dried preparation includes the above-mentioned anti-CD20 ADC, histidine hydrochloride, trehalose, mannitol, and polysorbate 80.

Preferably, the anti-CD20 ADC is used in combination with at least one therapeutic agent, wherein the at least one therapeutic agent is selected from the alkylating agents; the alkylating agent is selected from one or more of Cyclophosphamide, Ifosfamide, Melphalan, Busulfan, Chlormethine, Chlorambucil, Thiotepa, Bendamustine, Carmustine, Nimustine, Lomustine, and Semustine.

Preferably, the at least one therapeutic agent is Bendamustine. Preferably, the NHL is R/R NHL.

Further, the R/R NHL is R/R NHL after receiving at least one standard treatment regimen.

Preferably, the NHL is selected from small lymphocytic lymphoma (SLL), chronic lymphocytic leukemia (CLL), marginal zone lymphoma (MZL), follicular lymphoma (FL), diffuse large B-cell lymphoma (DLBCL), Burkitt lymphoma (BL), mantle cell lymphoma (MCL), Precursor B-cell lymphoblastic leukemia/lymphoma (B-ALL/LBL), T-cell large granular lymphocytic leukemia (T-LGLL), or anaplastic large cell lymphoma (ALCL).

Preferably, the NHL is DLBCL.

Preferably, the DLBCL is R/R DLBCL.

Further, the DLBCL is R/R DLBCL after receiving at least one standard treatment regimen.

Further, the DLBCL is CD20-positive DLBCL.

Further, the CD20-positive DLBCL is DLBCL with dual expression of MYC and BCL2.

Furthermore, the CD20-positive DLBCL is DLBCL with high or low CD20 expression.

Furthermore, the CD20-positive DLBCL is DLBCL with high CD20 expression and high expression of one or more of MYC, BCL2, and BCL6. Furthermore, the CD20-positive DLBCL is DLBCL with high CD20 expression and high expression of MYC and BCL2.

Furthermore, the CD20-positive DLBCL is DLBCL with low CD20 expression and high expression of one or more of MYC, BCL2, and BCL6. Furthermore, the CD20-positive DLBCL is DLBCL with low CD20 expression and high expression of MYC and BCL2.

Preferably, the at least one standard treatment regimen includes at least anti-CD20 antibody monotherapy or anti-CD20 antibody combination therapy.

Further, the at least one standard treatment regimen includes at least one selected from the group consisting of R-CHOP, R-CHOEP, R-miniCHOP, DA-EPOCH-R, and Pola-R-CHP.

Furthermore, the at least one standard treatment regimen is R-CHOP.

**A second aspect of the present disclosure provides a drug combination for treating NHL.**

The drug combination comprises an anti-CD20 ADC and at least one therapeutic agent, and the anti-CD20 ADC has the following structure:

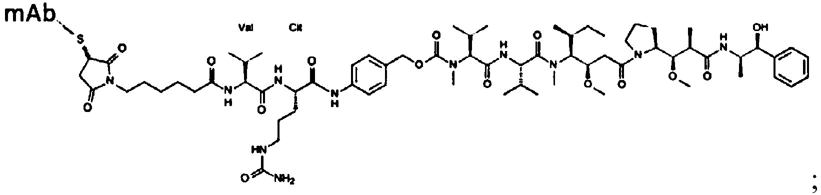

Wherein mAb represents a recombinant anti-CD20 mAb with a DAR of 1 to 8; preferably, DAR is 4.2 ± 1; further preferably, DAR is 4.2 ± 0.5; and still more preferably, DAR is 4.2 ± 0.3.

Preferably, the recombinant anti-CD20 mAb is selected from Rituximab or a biosimilar thereof, Ofatumumab or a biosimilar thereof, Ocrelizumab or a biosimilar thereof, or Obinutuzumab or a biosimilar thereof.

Further preferably, the recombinant anti-CD20 mAb is selected from Rituximab or a biosimilar thereof.

The at least one therapeutic agent is an alkylating agent; the alkylating agent is selected from one or more of Cyclophosphamide, Ifosfamide, Melphalan, Busulfan, Chlormethine, Chlorambucil, Thiotepa, Bendamustine, Carmustine, Nimustine, Lomustine, and Semustine. Preferably, the at least one therapeutic agent is Bendamustine. Preferably, the drug combination described in the present disclosure is a combination of the anti-CD20 ADC and Bendamustine.

The above-mentioned combination of the anti-CD20 ADC and at least one therapeutic agent is only an exemplary combination and does not constitute a limitation on the use of the anti-CD20 ADC of the present disclosure in combination with other drugs.

The drug combination described in the present disclosure is composed of a first pharmaceutical composition comprising the anti-CD20 ADC and a carrier or excipient thereof, and a second pharmaceutical composition comprising at least one therapeutic agent and a carrier or excipient thereof. The first pharmaceutical composition and the second pharmaceutical composition described in the present disclosure can be packaged separately or co-packaged.

The present disclosure also discloses a kit, which contains the anti-CD20 ADC and the at least one therapeutic agent.

Preferably, the anti-CD20 ADC and the at least one therapeutic agent are separately packaged and arranged in the kit.

Preferably, the kit contains the anti-CD20 ADC and Bendamustine.

**A third aspect of the present disclosure provides a method for treating NHL.**

The combination therapy described in the present disclosure comprises administering an effective dose of the drug combination disclosed in the second aspect of the present disclosure or the above-mentioned anti-CD20 ADC for treating NHL.

Preferably, the combination therapy comprises co-administering an effective dose of the anti-CD20 ADC and Bendamustine.

Preferably, the NHL is R/R NHL.

Further, the R/R NHL is R/R NHL after receiving at least one standard treatment regimen.

Preferably, the NHL is selected from small lymphocytic lymphoma (SLL), chronic lymphocytic leukemia (CLL), marginal zone lymphoma (MZL), follicular lymphoma (FL), diffuse large B-cell lymphoma (DLBCL), Burkitt lymphoma (BL), mantle cell lymphoma (MCL), Precursor B-cell lymphoblastic leukemia/lymphoma (B-ALL/LBL), T-cell large granular lymphocytic leukemia (T-LGLL), or anaplastic large cell lymphoma (ALCL).

Preferably, the NHL is DLBCL.

Preferably, the DLBCL is R/R DLBCL.

Further, the DLBCL is R/R DLBCL after receiving at least one standard treatment regimen.

Further, the DLBCL is CD20-positive DLBCL.

Further, the CD20-positive DLBCL is DLBCL with dual expression of MYC and BCL2.

Furthermore, the CD20-positive DLBCL is DLBCL with high or low CD20 expression.

Furthermore, the CD20-positive DLBCL is DLBCL with high CD20 expression and high expression of one or more of MYC, BCL2, and BCL6.

Furthermore, the CD20-positive DLBCL is DLBCL with high CD20 expression and high expression of MYC and BCL2.

Furthermore, the CD20-positive DLBCL is DLBCL with low CD20 expression and high expression of one or more of MYC, BCL2, and BCL6.

Furthermore, the CD20-positive DLBCL is DLBCL with low CD20 expression and high expression of MYC and BCL2.

Preferably, the at least one standard treatment regimen includes at least anti-CD20 antibody monotherapy or anti-CD20 antibody combination therapy.

Further, the at least one standard treatment regimen includes at least one selected from the group consisting of R-CHOP, R-CHOEP, R-miniCHOP, DA-EPOCH-R, and Pola-R-CHP.

Furthermore, the at least one standard treatment regimen is R-CHOP. The anti-CD20 ADC can be administered before, simultaneously with, and/or after the administration of the at least one therapeutic agent.

The anti-CD20 ADC and the at least one therapeutic agent can be administered once or multiple times. For example, the anti-CD20 ADC can be administered once or multiple times. In cases of multiple administrations, the frequency may vary, such as four times a week, three times a week, BIW, once a week, Q2W, Q3W, Q4W, Q5W, Q6W, Q7W, or Q8W. Alternatively, administration can follow a cycle-based schedule, such as intervals of one day, one week, two weeks, three weeks, four weeks, one month, two months, three months, and six months, with the drug administered once, twice, three times, four times, five times, and six times per cycle. Treatment continues based on the appropriate administration schedule and frequency until recovered, progressive disease (PD), or death. The at least one therapeutic agent, such as Bendamustine, can be administered once or multiple times. In cases of multiple administrations, the frequency may vary, such as TID, BID, QD, QOD, QW, Q2W, Q3W, or Q4W. Alternatively, administration can follow a cycle-based schedule, such as intervals of one day, one week, two weeks, three weeks, four weeks, one month, two months, three months, and six months, with the drug administered once, twice, three times, four times, five times, and six times per cycle. Treatment continues based on the appropriate administration schedule and frequency until recovered, PD, or death.

Depending on the type and severity of the disease, the initial candidate dose of the anti-CD20 ADC administered to the patient may be ~1 µg/kg to 100 mg/kg, and an exemplary dose of the anti-CD20 ADC may be 0.01 mg/kg to 50 mg/kg; preferably, ~0.05 mg/kg to ~20 mg/kg; preferably, ~0.1 mg/kg to ~10 mg/kg; preferably, ~0.5 mg/kg to ~5.0 mg/kg; and preferably, 0.5 mg/kg, 0.6 mg/kg, 0.75 mg/kg, 0.8 mg/kg, 1.0 mg/kg, 1.2 mg/kg, 1.5 mg/kg, 1.8 mg/kg, 2.0 mg/kg, and 2.5 mg/kg.

Depending on the type and severity of the disease, the dose of at least one therapeutic agent administered to the patient may be 1 mg/m² to 100 g/m² or 1 µg/kg to 100 mg/kg. For example, the dose of Bendamustine is 0.1 mg/m² to 10 g/m² or 0.1 µg/kg to 10 mg/kg; preferably, 1 mg/m² to 10 g/m²; preferably, 10 mg/m² to 1000 mg/m²; preferably, 20 mg/m² to 500 mg/m²; and preferably, 25 mg/m², 50 mg/m², 75 mg/m², 100 mg/m², 150 mg/m², 200 mg/m², and 250 mg/m².

The combined drug of the present disclosure can be administered by any suitable means, including gastrointestinal, parenteral, pulmonary, and intranasal. Parenteral infusion includes intramuscular, intravenous, intraarterial, intraperitoneal, or subcutaneous administration.

**A fourth aspect of the present disclosure provides a growth inhibition method for NHL cells.**

The growth inhibition method comprises adding an effective dose of the anti-CD20 ADC described in the present disclosure or the drug combination described in the second aspect of the present disclosure to the NHL cell system.

The growth inhibition method includes adding an effective dose of an anti-CD20 ADC to the NHL cell system, and the anti-CD20 ADC has the following structure:

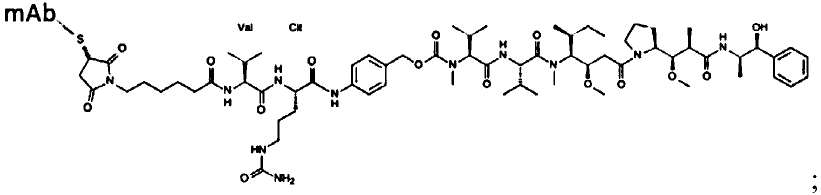

Wherein mAb represents a recombinant anti-CD20 mAb with a DAR of 1 to 8; preferably, DAR is 4.2 ± 1; further preferably, DAR is 4.2 ± 0.5; and still more preferably, DAR is 4.2 ± 0.3.

Preferably, the recombinant anti-CD20 mAb is selected from Rituximab or a biosimilar thereof.

Preferably, the DAR is 4.2 ± 0.5.

Alternatively, the growth inhibition method includes administering an effective dose of the drug combination described in the second aspect of the present disclosure to the NHL cell system.

Preferably, the growth inhibition method comprises administering an effective dose of the anti-CD20 ADC and Bendamustine to the NHL cell system.

Preferably, the NHL is R/R NHL.

Further, the R/R NHL is R/R NHL after receiving at least one standard treatment regimen.

Preferably, the NHL is selected from small lymphocytic lymphoma (SLL), chronic lymphocytic leukemia (CLL), marginal zone lymphoma (MZL), follicular lymphoma (FL), diffuse large B-cell lymphoma (DLBCL), Burkitt lymphoma (BL), mantle cell lymphoma (MCL), Precursor B-cell lymphoblastic leukemia/lymphoma (B-ALL/LBL), T-cell large granular lymphocytic leukemia (T-LGLL), or anaplastic large cell lymphoma (ALCL).

Preferably, the NHL is DLBCL.

Preferably, the DLBCL is R/R DLBCL.

Further, the DLBCL is R/R DLBCL after receiving at least one standard treatment regimen.

Further, the DLBCL is CD20-positive DLBCL.

Further, the CD20-positive DLBCL is DLBCL with dual expression of MYC and BCL2.

Furthermore, the CD20-positive DLBCL is DLBCL with high or low CD20 expression.

Furthermore, the CD20-positive DLBCL is DLBCL with high CD20 expression and high expression of one or more of MYC, BCL2, and BCL6. Furthermore, the CD20-positive DLBCL is DLBCL with high CD20 expression and high expression of MYC and BCL2.

Furthermore, the CD20-positive DLBCL is DLBCL with low CD20 expression and high expression of one or more of MYC, BCL2, and BCL6. Furthermore, the CD20-positive DLBCL is DLBCL with low CD20 expression and high expression of MYC and BCL2.

Preferably, the at least one inhibition treatment includes at least an anti-CD20 antibody monotherapy or an anti-CD20 antibody combined with at least one therapeutic agent.

Further, the at least one inhibition treatment includes at least one selected from the group consisting of R-CHOP, R-CHOEP, R-miniCHOP, DA-EPOCH-R, and Pola-R-CHP.

Further, the at least one inhibition treatment is R-CHOP.

Based on actual clinical needs, the present disclosure takes the clinical standard treatment regimen as a control. For NHL, especially CD20-positive DLBCL with dual expression of BCL2 and MYC, the anti-CD20 ADC of the present disclosure, used alone or in combination with the Bendamustine regimen, shows better tumor inhibition effect and better treatment potential than the existing second-line standard therapy, and has good safety.

It is to be understood that one, some, or all of the properties of the various embodiments described herein may be combined to form other embodiments of the present disclosure. Specific embodiments of the present disclosure are further described in detail as follows.

### BRIEF DESCRIPTION OF DRAWINGS

Embodiments of the present disclosure are described in detail below with reference to the attached figures, wherein:
Figure 1: Tumor Volume Change Trend of Mice in Each Treatment Group in WSU-DLCL2 Model;
Figure 2: Tumor Weight of Mice in Each Treatment Group at the End of the Study in WSU-DLCL2 Model; and
Figure 3: Tumor Volume Change Trend of Mice in Each Treatment Group in OCI-LY19 Model.

### DESCRIPTION OF EMBODIMENTS

The present disclosure is described below with reference to specific examples. It will be appreciated by those skilled in the art that these examples are for illustration of the disclosure only and are not intended to limit the scope of the present disclosure in any way.

The experimental methods in the following examples are conventional methods unless otherwise specified. The raw materials, reagent materials, and the like used in the examples described below are commercially available products unless otherwise specified.

### Definition:

"Rituximab" refers to a monoclonal antibody drug targeting CD20 developed by Roche and approved for marketing by the National Medical Products Administration (NMPA) under the trade name of "MabThera". Rituximab is the generic name, and only the innovator drug can be called Rituximab.

"Rituximab biosimilar" refers to the monoclonal antibody drug developed by other companies in addition to the innovator drug of Rituximab, with identical amino acid sequences and comparable physical and chemical properties, efficacy, pharmacokinetics, and safety to the innovator drug.

"High expression" refers to cases where the test results for antigen or gene expression levels in tumor tissues or cells exceed a certain threshold or are relatively high based on conventional evaluation criteria. For instance, high antigen or gene expression may be identified when immunohistochemical staining indicates strong positivity (+++) and/or positivity (++), as determined by the proportion of stained cells and staining intensity.

"Low expression" refers to cases where the test results for antigen or gene expression levels in tumor tissues or cells are below a certain threshold or are relatively low based on conventional evaluation criteria. For instance, low antigen or gene expression may be identified when immunohistochemical staining indicates weak positivity (+), as determined by the proportion of stained cells and staining intensity.

"Effective dose" refers to the amount that effectively achieves the desired treatment result at the required dose and time period. The effective dose may vary depending on factors such as the individual's disease state, age, gender, and weight, and the ability of the therapeutic agent or combination of therapeutic agents to induce a desired response in the individual.

Although the present disclosure has been generally described, examples of the present disclosure will be further disclosed in the following examples, which should not be construed as limiting the scope of the claims.

### Example 1: Preparation of Anti-CD20 ADC (TRS005)

An exemplary preparation method of an anti-CD20 ADC (TRS005) is specifically shown in Chinese Patent CN108452318B, the entire contents of which are incorporated in the present disclosure.

The structure of TRS005 is as follows: 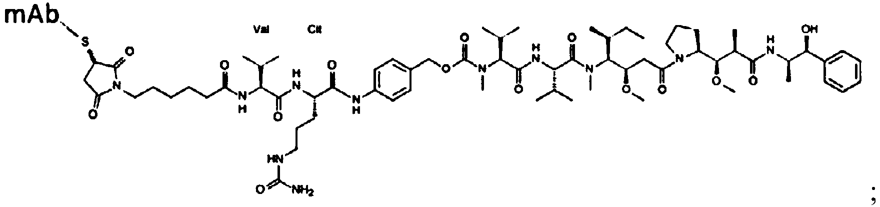 wherein mAb is Rituximab or a biosimilar thereof, and the DAR is 4.2 ± 0.5.

### Example 2: Screening of Human Lymphoma Cell Lines

By sequencing and detecting the expression levels (mRNA levels) of BCL2, MYC, and CD20 in each lymphoma cell line (SU-DHL-4, SU-DHL-6, TMD-8, WSU DLCL2, DOHH2, DB, OCI-LY10, and OCI-LY19), the lymphoma cell line WSU-DLCL2 with dual expression of BCL2 and MYC and high CD20 expression, and the lymphoma cell line OCI-LY19 with dual expression of BCL2 and MYC and low CD20 expression were screened out. WSU-DLCL2 and OCI-LY19 are CD20-positive cell lines with dual expression of BCL2 and MYC. The expression levels of CD20, BCL2, and MYC in the two cell lines are shown in Table 1.

**Table 1: Expression Levels of CD20, BCL2, and MYC in Two Cell Lines (log₂TPM)**

| | WSU DLCL2 | OCI-LY19 |
|---|---|---|
| CD20 | 9.360825 | 3.690417 |
| BCL2 | 6.178316 | 5.953964 |
| MYC | 7.047015 | 9.049576 |

### Example 3: Anti-tumor Efficacy Evaluation of TRS005 Alone or in Combination with Bendamustine in Human Lymphoma WSU-DLCL2/R-CHOP Relapsed Model

### (I) Model Establishment

The relapsed model is established using conventional operational methods in the art, wherein WSU-DLCL2 cells are inoculated into mice and treated with R-CHOP, defined as P1; tumor tissues with favorable growth status from WSU-DLCL2/R-CHOP (P1) are selected, and after removing the murine fascia and internal necrotic tissues, the tumor fragments are inoculated into mice under aseptic conditions and treated with R-CHOP, defined as P2; tumor tissues with favorable growth status from WSU-DLCL2/R-CHOP (P2) are selected, and after removing the murine fascia and internal necrotic tissues, the tumor fragments are inoculated into mice under aseptic conditions and treated with R-CHOP, defined as P3. Through R-CHOP treatment and serial passaging from P1 to P3, the R-CHOP relapsed model is established.

Select tumor tissues with favorable growth status from WSU-DLCL2/R-CHOP (P3), remove the murine fascia and internal necrotic tissues, cut into 2 × 2 × 2 mm³ small fragments, and store them in RPMI 1640 basal medium for later use. After the animals are anesthetized with isoflurane, tissue fragments are inoculated subcutaneously on the right side of the mice using an 11G cannula needle under sterile conditions.

### (II) Grouping and Administration

When the mean tumor volume (TV) reaches ~150 mm³, the animals are randomly divided into groups according to the TV so that the difference in TV between each group is < 10% of the mean. In this study, there were 6 animals in each group. The day of grouping was recorded as Day 0, and the drug was administered immediately according to the animal body weight (BW) after grouping. On Day 24, the remaining animals were sacrificed, the tumors were weighed and photographed for recording, and then the study ended. The grouping and administration regimen are shown in Table 2 below.

**Table 2 Grouping and Administration Regimen**

| **Group** | **Treatment** | | **Number of Animals** | **Dose (mg/kg)** | **Route of Administration** | **Administration Schedule** |
|---|---|---|---|---|---|---|
| 1 | Vehicle | Vehicle | 6 | / | IV | QWx3w |
| 2 | R+Bendamustine | Bendamustine | 6 | 2.5 | IV | BIWx3w |
| | | MabThera | | 10 | IV | BIWx3w |
| 3 | TRS005 | TRS005 | 6 | 1.5 | IV | BIWx3w |
| 4 | TRS005+Bendamustine | TRS005 | 6 | 1.5 | IV | BIWx3w |
| | | Bendamustine | | 2.5 | IV | BIWx3w |

Wherein, IV: intravenous injection; QWx3w: once a week for three weeks; BIWx3w: twice a week for three weeks.

The BW and TV of animals were measured twice a week during the study. During the study, the clinical symptoms of animals were observed and recorded once a day. Clinical observations included general health status, abnormal BW and behavior of animals, and other treatment-related adverse reactions.

### (III) Evaluation Indicators and Statistical Analysis

The tumor growth inhibition rate (%TGI_{TV}) is calculated as follows: (1 - TV_{T} / TV_{C}) × 100%, where TV_{C} is the mean TV of the negative control group, and TV_{T} is the mean TV of the treatment group.

Relative Tumor Volume (RTV) is calculated as Vt / V0, where V0 is the TV at grouping and Vt is the TV at each measurement.

The calculation formula for relative tumor proliferation rate (%T/C_{RTV}) is: T_{RTV} / C_{RTV} × 100%, where T_{RTV} is the RTV of the treatment group and C_{RTV} is the RTV of the negative control group.

The %TGI_{TW} is calculated as follows: (TW_{C} - TW_{T}) / TW_{C} × 100%, where TW_{C} is the mean TW of the negative control group and TW_{T} is the mean TW of the treatment group.

The animal body weight change (%BWC) is calculated as follows: (BWt - BW0) / BW0 × 100%, where BWt is the animal BW at each measurement and BW0 is the animal BW at grouping.

According to the *Technical Guidelines for Nonclinical Study of Cytotoxic Anti-tumor Drugs* (November 2006) issued by NMPA in China, it is considered effective if %T/C_{RTV} ≤ 40% and *P* < 0.05 after statistical analysis. Drug doses are considered to be severely toxic when the number of animal deaths related to the drug exceeds 20%.

The study data are expressed as Mean ± SEM, and a two-tailed t-test is used for intergroup comparisons. *P* < 0.05 is a significant difference, and *P* < 0.01 is an extremely significant difference (Microsoft Excel 2007, Redmond, WA, USA).

### (IV) Study Results

### 1. Tumor Inhibition Effect of Test Article

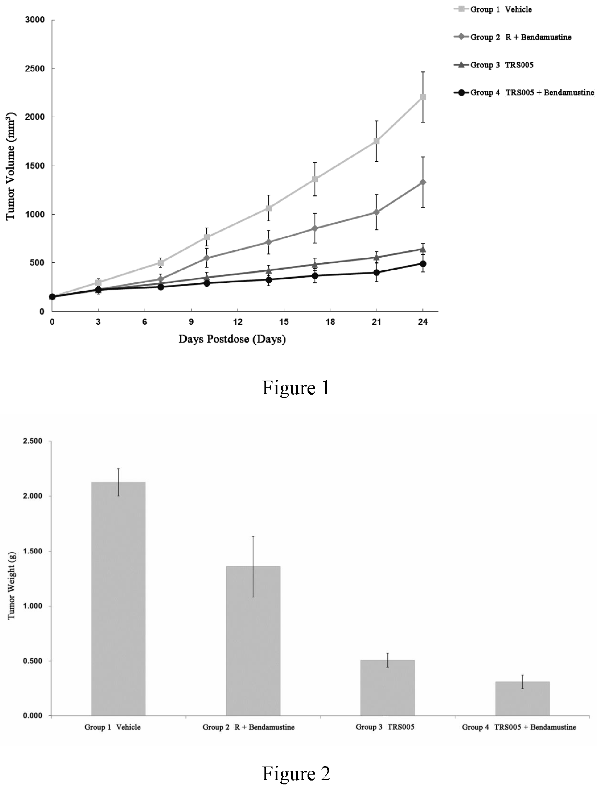

In this study, the study results of anti-tumor activity on TRS005 alone or in combination with Bendamustine in the human lymphoma WSU-DLCL2/R-CHOP relapsed model are shown in Tables 3 and 4 and Figures 1 and 2.

As shown in Table 3 and Figures 1 and 2, on Day 24, the TW of Group 4 (TRS005 + Bendamustine) was 0.311 ± 0.062 g, with %TGI of 85.36%; the TW of Group 2 (R + Bendamustine) was 1.359 ± 0.277 g, with %TGI of 36.05%; the TW of Group 3 (TRS005) was 0.508 ± 0.063 g, with %TGI of 76.09%.

**Table 3 Anti-tumor Activity of TRS005 Monotherapy or in Combination with Bendamustine**

| **Group** | **Treatment** | **Tumor Volume (mm³, Mean±SEM)** | | **Tumor Weight (g) Day 24** | **%TGI_{TW} Day 24** | **RTV Day 24** | **%T/C_{RTV} Day 24** |
|---|---|---|---|---|---|---|---|
| | | **Day 0** | **Day 24** | | | | |
| 1 | Vehicle | 151.49±22.29 | 1064.14 ± 132.2 (Day 14) | 2.125±0.124 | / | 7.86 ± 1.89 (Day 14) | / |
| | | | 2204.81 ±260.81 (Day 24) | | | 15.80 ±2.95 (Day 24) | |
| 2 | R + Bendamustine | 152.37±19.03 | 1330.79 ±258.63 * | 1.359 ±0.277 * | 36.05 | 8.36 ± 0.79 * | 52.91 |
| 3 | TRS005 | 151.92±18.56 | 642.09 ± 56.07 *** | 0.508 ± 0.063 *** | 76.09 | 4.41 ± 0.43 ** | 27.91 |
| 4 | TRS005 + Bendamustine | 151.60±12.01 | 494.21 ± 87.04 *** | 0.311 ± 0.062 *** | 85.36 | 3.14 ±0.40 ** | 19.87 |

| | | | | | | | |
|---|---|---|---|---|---|---|---|
| *: *P* < 0.05 vs. Vehicle Group **: *P* < 0.01 vs. Vehicle Group ***: *P* < 0.001 vs. Vehicle Group | | | | | | | |

As shown in Table 4, the significance analysis of tumor inhibition activity showed that the %TGI of Group 4 (TRS005 + Bendamustine) was significantly higher than that of Group 2 (R + Bendamustine) and Group 3 (TRS005), achieving unexpected technical effects; the %TGI of Group 3 (TRS005) was significantly higher than that of Group 2 (R + Bendamustine), achieving unexpected technical effects.

**Table 4 Significance Analysis of Anti-tumor Activity of TRS005 Monotherapy or in Combination with Bendamustine**

| **Group** | **Significant (*P* Value)** | |
|---|---|---|
| | Group 2 (R+Bendamustine) | Group 3 (TRS005) |
| Group 4 (TRS005 + Bendamustine) | 0.004 | 0.049 |
| | *P*<0.01 | *P*<0.05 |
| Group 2 (R + Bendamustine) | / | 0.013 |
| | | *P*<0.05 |

### 2. Effect of Test Article on Body Weight of Tumor-bearing Animals

The results of the effect of each test article on the BW of WSU-DLCL2 tumor-bearing animals showed that on Day 24, the mean BW of animals in each group increased by 0.8% to 5.99% (-0.17 g to 1.30 g) compared with the start of the study on Day 0. The effect of treatment on the BW of tumor-bearing animals in each group is shown in Table 5.

**Table 5 Effect of Treatment on Body Weight of Tumor-bearing Animals in Each Group**

| **Group** | **Treatment** | **Animal Body Weight (g, Mean±SEM)** | | **%BWC Day 24** | **Number of Mice with Drug-related Deaths Day 25** |
|---|---|---|---|---|---|
| | | **Day 0** | **Day 24** | | |
| 1 | Vehicle | 21.72±0.23 | 23.02±0.53 | 5.95 (1.3g) | 0/6 |
| 2 | R+Bendamustine | 20.89±0.35 | 21.60±0.85 | 3.25 (0.71g) | 0/6 |
| 3 | TRS005 | 21.52±0.35 | 21.97±0.44 | 2.17 | 0/6 |
| | | | | (0.45g) | |
| 4 | TRS005 + Bendamustine | 21.24±0.4 | 21.41±0.76 | 0.67 (0.17g) | 0/6 |

During the study, the body weight change (BWC) of mice in each treatment group was within the normal range, no drug-related mouse deaths occurred, and no other obvious drug-related toxicity was observed, indicating that the TRS005 monotherapy and TRS005 combination administration regimen of the present disclosure have good safety.

### Example 4: Anti-tumor Efficacy Evaluation of TRS005 with Bendamustine in Human Lymphoma OCI-LY19 Model

### (I) Grouping and Administration

When the mean TV reaches 100 to 120 mm³, the animals are randomly divided into groups according to the TV so that the difference in TV between each group is < 10% of the mean. The day of grouping was recorded as Day 0, and the administration was started according to animal BW. The grouping and administration regimen are shown in Table 6.

**Table 6 Grouping and Administration Regimen**

| **Group** | **Treatment** | | **Number of Animals** | **Dose (mg/kg)** | **Route of Administration** | **Administration Schedule** |
|---|---|---|---|---|---|---|
| 1 | Vehicle | Vehicle | 10 | / | IV | QWx3wks |
| 2 | R+Bendamustine | MabThera | 10 | 10 | IV | BIWx3wks |
| | | Bendamustine | | 3 | IV | BIWx3wks |
| 3 | TRS005 + Bendamustine | TRS005 | 10 | 1.5 | IV | BIWx3wks |
| | | Bendamustine | | 3 | IV | BIWx3wks |

Wherein, IV: intravenous injection; QW × 3wks: once a week for three weeks; BIW × 3wks: twice a week for three weeks.

During the study, BW and TV of animals were measured twice a week, and clinical symptoms of animals were observed and recorded once a day. Animals with TV > 2000 mm³ were euthanized by CO₂ after the last weighing, and the remaining animals in the same group continued to be administered and observed until the animal welfare endpoint or study endpoint was reached. The study period was 28 days (3 weeks of drug administration and 1 week of observation after drug withdrawal). After the last weighing at the end of the study, the remaining animals were euthanized by CO₂, the tumors were taken and weighed, and then the study ended.

### (II) Study Result

### 1. Tumor Inhibition Effect of Test Article

The study period was 28 days. On Day 18, mice in the Vehicle Group began to have TV > 2000 mm³ and were euthanized at the end of the study. Since the TV data of the Vehicle Group is required in the calculation of %TGI_{TV}, the data on Day 18 are used for statistical analysis.

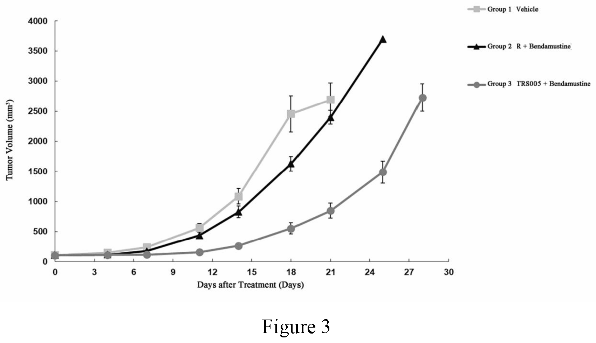

As shown in Table 7 and Figure 3, in the study on anti-tumor activity of TRS005 + Bendamustine in the human lymphoma OCI-LY19 model, on Day 18, the mean TV of Group 2 (R + Bendamustine) was 1628.64 ± 121.69 mm³, with %TGI_{TV} of 33.68%; the mean TV of Group 3 (TRS005 + Bendamustine) was 555.50 ± 95.66 mm³, with %TGI_{TV} of 77.38%. R + Bendamustine is the second-line clinical standard therapy for DLBCL. Significance analysis shows that, compared with R + Bendamustine, TRS005 + Bendamustine has a significantly improved tumor inhibition effect and achieved unexpected technical effects.

The last dose was administered on Day 14. As shown in Figure 3, after Day 18, the TV of mice in Group 2 (R + Bendamustine) increased rapidly. Although the TV of mice in Group 3 (TRS005 + Bendamustine) also increased, the growth rate was lower than that in Group 2. On Day 21, 9 mice in Group 2 had TVs reaching the euthanasia standard and were euthanized, while no mice in Group 4 were euthanized; on Day 25, the last mouse in Group 2 was euthanized, while only one mouse in Group 4 had a TV reaching the euthanasia standard, further indicating that the tumor inhibition effect of TRS005 + Bendamustine in Group 4 was superior to that of R + Bendamustine in Group 2.

**Table 7 Anti-tumor Activity of TRS005 in Combination with Bendamustine**

| **Group** | **Treatment** | **Tumor Volume (mm³, Mean±SEM)** | | **%TGI_{TV} Day 18** | **RTV Day 18** | **%T/C_{RTV} Day 18** |
|---|---|---|---|---|---|---|
| | | **Day 0** | **Day 18** | | | |
| 1 | Vehicle | 107.80±3.63 | 2455.69±298.16 | / | 22.91±2.77 | / |
| 2 | R+Bendamustine | 107.47±3.28 | 1628.64±121.69 * | 33.68 | 15.21±1.13 | 66.39 |
| 3 | TRS005 + Bendamustine | 106.37±2.62 | 555.50±95.66 ***### | 77.38 | 5.22±0.88 | 22.78 |

| | | | | | | |
|---|---|---|---|---|---|---|
| *: *P* < 0.05 vs. Vehicle Group **: *P* < 0.01 vs. Vehicle Group ***: *P* < 0.001 vs. Vehicle Group #: *P* < 0.05 vs. R + Lenalidomide Group ##: *P* < 0.01 vs. R + Lenalidomide Group ###: *P* < 0.001 vs. R + Lenalidomide Group | | | | | | |

### 2. Effect of Test Article on Body Weight of Tumor-bearing Animals

The results of the effect of each test article on the BW of OCI-LY19 tumor-bearing animals showed that on Day 18, the mean BW of animals in each group increased by 5.35% to 13.07% (1.18 g to 2.90 g) compared with the start of the study on Day 0. The effect of treatment on the BW of tumor-bearing animals in each group is shown in Table 8.

**Table 8 Effect of Treatment on Body Weight of Tumor-bearing Animals in Each Group**

| **Group** | **Number of Animals** | **Treatment** | **Animal Body Weight (g, Mean±SEM)** | | **%BWC Day 18** | **Number of Mice with Drug-related Deaths Day 28** |
|---|---|---|---|---|---|---|
| | | | **Day 0** | **Day 18** | | |
| 1 | 10 | Vehicle | 22.19±0.42 | 25.09±0.54 | 13.10 (2.90g) | 0/10 |
| 2 | 10 | R+Bendamustine | 22.32±0.28 | 24.37±0.36 | 9.25 (2.05g) | 0/10 |
| 3 | 10 | TRS005+Bendamustine | 22.04±0.41 | 23.22±0.49 | 5.37 (1.18g) | 0/10 |

During the study, the BWC of mice in each treatment group was within the normal range, no drug-related mouse deaths occurred, and no other obvious drug-related toxicity was observed, indicating that the TRS005 combined with Bendamustine regimen of the present disclosure has good safety.

In summary, to improve the actual clinical benefits of NHL patients, the present disclosure is rooted in addressing actual clinical needs and takes the clinical standard treatment regimen as a control. The TRS005 + Bendamustine regimen adopted by the present disclosure significantly improves the tumor inhibition effect compared with the second-line standard therapy (R + Bendamustine) and TRS005 monotherapy in NHL, especially CD20-positive DLBCL, and the TRS005 monotherapy of the present disclosure also significantly improves the tumor inhibition effect compared with the second-line standard therapy (R + Bendamustine). The above-mentioned results showed that the TRS005 monotherapy or in combination with Bendamustine regimen of the present disclosure exhibits better tumor inhibition effect and better treatment potential than the existing second-line standard therapy, and the TRS005 monotherapy or combined regimen of the present disclosure has good safety, which can better meet clinical treatment needs.

The above description of embodiments is not intended to limit the present disclosure. Those skilled in the art may make various changes or modifications according to the present disclosure, and such changes or modifications are intended to fall within the scope of the appended claims if they do not depart from the spirit of the disclosure.

## Claims

1. A use of an anti-CD20 antibody drug conjugate (ADC) for preparing a drug to treat Non-Hodgkin Lymphoma (NHL), wherein the anti-CD20 ADC has the following structure: 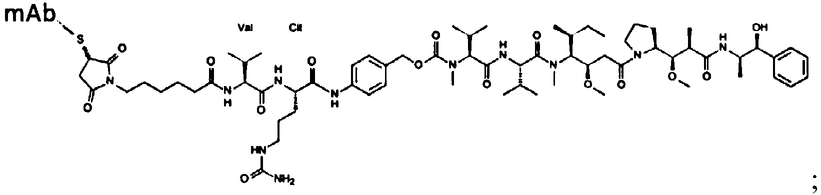 Wherein, mAb represents a recombinant anti-CD20 mAb with a drug antibody ratio (DAR) of 4.2 ± 1; preferably, DAR is 4.2 ± 0.5.

2. The use according to claim 1, wherein the Non-Hodgkin Lymphoma (NHL) is relapsed or refractory (R/R) NHL.

3. The use according to claim 2, wherein the NHL is R/R NHL after receiving at least one standard treatment regimen.

4. The use according to claim 3, wherein the recombinant anti-CD20 mAb is selected from Rituximab or a biosimilar thereof, Ofatumumab or a biosimilar thereof, Ocrelizumab or a biosimilar thereof, or Obinutuzumab or a biosimilar thereof.

5. The use according to claim 4, wherein the recombinant anti-CD20 mAb is selected from Rituximab or a biosimilar thereof.

6. The use according to any one of claims 1 to 5, wherein the NHL is selected from small lymphocytic lymphoma (SLL), chronic lymphocytic leukemia (CLL), marginal zone lymphoma (MZL), follicular lymphoma (FL), diffuse large B-cell lymphoma (DLBCL), Burkitt lymphoma (BL), mantle cell lymphoma (MCL), Precursor B-cell lymphoblastic leukemia/lymphoma (B-ALL/LBL), T-cell large granular lymphocytic leukemia (T-LGLL), or anaplastic large cell lymphoma (ALCL).

7. The use according to claim 6, wherein the NHL is diffuse large B-cell lymphoma (DLBCL).

8. The use according to claim 7, wherein the DLBCL is CD20-positive DLBCL.

9. The use according to claim 8, wherein the CD20-positive DLBCL is DLBCL with dual expression of MYC and BCL2.

10. The use according to any one of claims 1 to 9, wherein the at least one standard treatment regimen includes at least anti-CD20 antibody monotherapy or anti-CD20 antibody combination therapy.

11. The use according to claim 10, wherein the at least one standard treatment regimen includes at least one selected from the group consisting of R-CHOP, R-CHOEP, R-miniCHOP, DA-EPOCH-R, and Pola-R-CHP.

12. The use according to claim 11, wherein the at least one standard treatment regimen is R-CHOP.

13. The use according to any one of claims 1 to 12, wherein the anti-CD20 ADC is used in combination with Bendamustine.

14. A drug combination for treating NHL, wherein the drug combination is a combination of an anti-CD20 antibody drug conjugate (ADC) and Bendamustine, and the anti-CD20 ADC has the following structure: 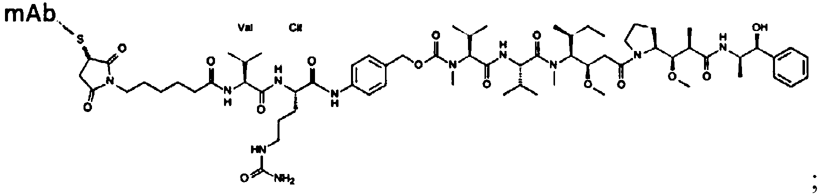 Wherein, mAb represents a recombinant anti-CD20 mAb with a DAR of 4.2 ± 1; preferably, DAR is 4.2 ± 0.5.

15. The drug combination according to claim 14, wherein the NHL is R/R NHL.

16. The drug combination according to claim 15, wherein the NHL is R/R NHL after receiving at least one standard treatment regimen.

17. The drug combination according to any one of claims 14 to 16, wherein the recombinant anti-CD20 mAb is selected from Rituximab or a biosimilar thereof, Ofatumumab or a biosimilar thereof, Ocrelizumab or a biosimilar thereof, or Obinutuzumab or a biosimilar thereof.

18. The drug combination according to claim 17, wherein the recombinant anti-CD20 mAb is selected from Rituximab or a biosimilar thereof.

19. A method for treating NHL, wherein an effective dose of the drug combination according to any one of claims 14 to 18 or the anti-CD20 ADC according to any one of claims 14 to 18 is administered to a subject.

20. The combination therapy according to claim 19, wherein the NHL is selected from small lymphocytic lymphoma (SLL), chronic lymphocytic leukemia (CLL), marginal zone lymphoma (MZL), follicular lymphoma (FL), diffuse large B-cell lymphoma (DLBCL), Burkitt lymphoma (BL), mantle cell lymphoma (MCL), precursor B-cell lymphoblastic leukemia/lymphoma (B-ALL/LBL), T-cell large granular lymphocytic leukemia (T-LGLL), or anaplastic large cell lymphoma (ALCL).

21. The combination therapy according to claim 20, wherein the NHL is DLBCL.

22. The combination therapy according to claim 21, wherein the DLBCL is CD20-positive DLBCL.

23. The combination therapy according to claim 22, wherein the CD20-positive DLBCL is DLBCL with dual expression of MYC and BCL2.

24. The combination therapy according to any one of claims 19 to 23, wherein the at least one standard treatment regimen includes at least anti-CD20 antibody monotherapy or anti-CD20 antibody combination therapy.

25. The combination therapy according to claim 24, wherein the at least one standard treatment regimen includes at least one selected from the group consisting of R-CHOP, R-CHOEP, R-miniCHOP, DA-EPOCH-R, and Pola-R-CHP.

26. The combination therapy according to claim 25, wherein the at least one standard treatment regimen is R-CHOP.
